# FASCICULE DE BREVET EUROPEEN

(11) **EP 1 277 436 B1**
(45) Date de publication et mention de la délivrance du brevet: **02.12.2009**
(21) Numéro de dépôt: 02291574.8
(22) Date de dépôt: 25.06.2002
(51) Int. Cl.: A61B 5/103

(54) **Instrument pour observer la peau ou les cheveux**
Instrument zur Beobachtung der Haut oder der Haare
Instrument to observe the skin or hairs

(30) Priorité: 09.07.2001 FR 0109091
(43) Date de publication de la demande: 22.01.2003
(73) Titulaire: L'ORÉAL, 75008 Paris (FR)
(72) Inventeur: Bazin, Roland, 91570 Bievres (FR); Giron, Franck, 77164 Ferrieres-en-Brie (FR)
(74) Mandataire: Tanty, François

(56) Documents cités:
- WO-A-01/45557
- WO-A-96/16698
- FR-A- 2 650 890

## Description

La présente invention est relative à l'évaluation de caractéristiques de la typologique corporelle, notamment des caractéristiques d'apparence telles que la brillance, la couleur et le relief cutané, par exemple.

Il existe des dermatoscopes qui contiennent un système de grossissement et des moyens d'éclairage intégrés permettant de faire des observations précises. Toutefois, ces dermatoscopes ne sont pas prévus pour délivrer une image autre qu'un simple agrandissement de la zone observée. De plus, les caractéristiques de l'éclairage sont susceptibles de varier d'un dermatoscope à l'autre, ce qui n'est pas trop gênant lorsqu'il s'agit d'observer un défaut de la peau mais n'est pas satisfaisant lorsqu'il s'agit d'évaluer des caractéristiques d'apparence telles que la brillance ou la couleur, par exemple.

Des systèmes complexes utilisant des caméras vidéo ou d'autres capteurs électroniques existent par ailleurs, étant décrits par exemple dans les demandes de brevet WO 96 16698, FR 2650890, EP-A-0 655 221 ou les brevets US 5 377 000 et US 4 911 544. Ces systèmes sont relativement coûteux et se prêtent mal à une large diffusion afin d'équiper par exemple tous les points de vente d'un produit ou de permettre au public de s'évaluer lui-même.

Il existe par conséquent un besoin pour bénéficier d'un instrument simple à utiliser, relativement peu coûteux, permettant d'évaluer au moins une caractéristique d'apparence de la peau ou des cheveux, par exemple des caractéristiques liées à l'éclat du teint ou à l'éclat des cheveux, telles que la brillance et la couleur.

L'invention vise notamment, selon un de ses aspects, à répondre à ce besoin.

Elle y parvient grâce au dispositif selon la revendication 1.

Des images peuvent être générées de manière à pouvoir être comparées entre elles, soit simultanément, soit successivement.

Lesdites images peuvent être générées successivement en agissant entre les deux observations sur un organe du dispositif permettant de faire varier une caractéristique autre que le grossissement ou l'intensité lumineuse de la source.

Lesdites images peuvent aussi être générées simultanément lorsque le dispositif est agencé pour produire simultanément, avec des éléments optiques, deux images différentes de la zone observée.

Lesdites images peuvent différer par exemple par l'orientation d'une direction de polarisation d'un analyseur, par le degré de directivité de la lumière incidente, par la direction d'incidence de la lumière incidente, par la couleur de la lumière, par le filtrage de la lumière provenant de la zone examinée, ou encore par une action mécanique exercée sur la zone examinée, par exemple une pression, un plissement ou un étirement.

Lesdites images peuvent être ou non générées pour suivre l'évolution d'une zone examinée au cours du temps.

Le dispositif d'observation peut permettre de suivre l'évolution d'une zone de peau au cours du temps, en générant à un instant donné au moins deux images de la zone de peau, différant autrement que par le grossissement ou l'intensité lumineuse d'une source intégrée, et en générant, par exemple un mois plus tard, au moins deux images de la même zone de peau, différant autrement que par le grossissement ou l'intensité lumineuse d'une source intégrée.

Le dispositif selon l'invention permet une observation directe, c'est-à-dire en regardant au travers du dispositif d'observation la zone examinée, sans acquisition par un appareil photographique, une caméra vidéo ou d'autres types de capteurs électroniques. Son coût de production peut ainsi rester compatible avec une large diffusion.

Par ailleurs, l'observation pouvant s'effectuer dans des conditions d'illumination prédéfinies, il est possible de quantifier la caractéristique observée d'une manière relativement précise et reproductible.

Dans une réalisation particulière, le dispositif comporte un système optique agencé pour produire une image agrandie de la zone examinée. Il est ainsi possible d'évaluer des caractéristiques de la peau difficiles à observer à l'oeil nu, telles que par exemple sa desquamation.

Toujours dans une réalisation particulière, le dispositif est agencé pour permettre de réduire ou d'éliminer la brillance d'une partie au moins de la zone examinée, ce qui peut permettre par exemple d'observer plus facilement la couleur de la peau ou des cheveux, sans être gêné par des reflets. La suppression ou la diminution de la brillance peut également être utile pour observer la couleur de la lumière qui est rétrodiffusée, provenant des couches profondes de l'épiderme ou des cheveux, et qui dépend de l'état de ces couches. Il peut notamment être intéressant d'observer le contraste entre la lumière qui comporte une composante de réflexion et une composante rétrodiffusée et la lumière qui comporte essentiellement une composante rétrodiffusée, en éclairant par exemple la zone examinée avec une lumière polarisée et en observant avec un analyseur. Ce dernier peut comporter un polariseur rotatif ou deux polariseurs ayant des directions de polarisation différentes, dont l'une peut être perpendiculaire à la direction de polarisation de la lumière incidente et l'autre parallèle.

Dans une réalisation particulière, la zone examinée peut être éclairée à l'aide d'un moyen d'éclairage permettant de l'illuminer avec des inclinaisons différentes de la lumière incidente, afin par exemple de l'observer sous éclairage diffus ou sous éclairage rasant.

On peut disposer ainsi de plusieurs conditions d'observation, ce qui peut permettre d'évaluer plus facilement une caractéristique d'apparence, en faisant ressortir les différences entre deux images, par exemple.

L'éclairage rasant peut donner des informations de relief et l'éclairage diffus des informations sur l'homogénéité de la couleur.

Dans une réalisation particulière, le dispositif comporte au moins un écran apte à être interposé entre une source de lumière et la zone examinée, de manière à ne permettre l'éclairage de cette dernière que par diffusion de la lumière sous l'écran, dans le tissu examiné. Cet écran peut être mobile, pouvant être déplacé d'une première position dans laquelle il est éloigné d'une surface bordant la zone examinée à une deuxième position dans laquelle il vient au contact de ladite surface. On peut ainsi observer la peau ou les cheveux par transillumination, c'est-à-dire que l'éclairage d'une zone de peau ou de cheveux s'effectue par la lumière provenant de zones adjacentes, la peau ou les cheveux servant de guide de lumière, et obtenir notamment une information de transparence, cette information pouvant se combiner le cas échéant à d'autres informations tirées d'observations précédentes ayant eu lieu dans des conditions d'illumination différentes. L'écran peut encore être amené dans une position dans laquelle il permet un éclairage rasant de la zone examinée. L'écran peut comporter une paroi tubulaire s'étendant, au cours de l'utilisation, autour de la zone examinée. L'écran, qui peut être de section non circulaire, peut comporter une portion conique ou pyramidale convergeant vers la zone examinée. Le dispositif peut comporter au moins un ressort pour rappeler l'écran dans une position de repos, en l'absence d'utilisation.

Le dispositif peut comporter un réticule, permettant notamment de mesurer la distance à partir de laquelle la diffusion de la lumière n'est plus visible, lorsque l'on observe par transillumination.

Dans une réalisation particulière, le dispositif comporte au moins un filtre coloré. Ce filtre peut être de couleur bleue, afin de faire ressortir la pigmentation de la peau, par exemple.

Dans une réalisation particulière, le dispositif comporte au moins un polariseur. Ce polariseur peut être placé sur le trajet de la lumière, entre une source de lumière et la zone examinée. Le dispositif peut également comporter au moins un polariseur placé sur le trajet de la lumière entre la zone examinée et un oeil de l'observateur. Le dispositif peut comporter au moins deux polariseurs d'orientations différentes, juxtaposés, placés sur le trajet de la lumière entre la zone examinée et un oeil de l'observateur. On peut ainsi observer un contraste entre deux zones de l'image, et profiter de la sensibilité plutôt élevée de l'oeil humain à un contraste. Le dispositif peut comporter au moins un polariseur monté rotatif de manière à permettre à l'utilisateur de modifier l'orientation de sa direction de polarisation par rapport à une direction de référence. Dans ce dernier cas, le dispositif peut comporter par exemple une poignée comportant un organe d'actionnement tel qu'une molette par exemple, permettant de modifier l'orientation du polariseur avec la même main que celle qui tient la poignée.

Le dispositif peut être agencé pour permettre l'éclairage de la zone examinée par la lumière naturelle. Le dispositif peut notamment comporter une jupe en matière plastique transparente, dont un bord peut s'étendre autour de la zone examinée.

Le dispositif peut aussi comporter au moins une source lumineuse intégrée. Cette source lumineuse intégrée peut comporter au moins un élément lumineux choisi parmi les suivants: lampe à incandescence, diode électroluminescente, lampe à fluorescence. Le dispositif peut comporter des éléments lumineux éclairant dans des gammes respectives de longueur d'onde différentes. Le dispositif peut par exemple comporter une source reproduisant les caractéristiques spectrales de la lumière naturelle, pouvant comporter des diodes électroluminescentes légèrement colorées. Le dispositif peut comporter plusieurs éléments lumineux et des moyens de commande permettant d'alimenter sélectivement au moins une partie de ces éléments lumineux. Le dispositif d'observation peut comporter des éléments lumineux disposés circulairement.

Le dispositif peut encore comporter un logement pour recevoir une ou plusieurs piles électriques. Un tel logement peut présenter un axe sensiblement perpendiculaire à une direction d'observation de la zone examinée.

Le dispositif peut comporter une vitre permettant de comprimer la peau dans la zone examinée pour en chasser le sang. Une telle vitre peut être réalisée en verre ou en matière plastique transparente, éventuellement colorée. Une telle vitre peut constituer un accessoire amovible ou un élément fixé à demeure sur le dispositif, mobile entre une position active dans laquelle elle s'interpose entre la peau et le trajet de la lumière vers l'observateur et une position inactive, dans laquelle la vitre ne se situe pas sur le trajet de la lumière vers l'observateur.

Le dispositif peut comporter une bague permettant l'adaptation d'un appareil photographique.

Dans une réalisation particulière, le dispositif est associé à un atlas comprenant une pluralité d'images de référence. Ces images peuvent être disposées sur un support unique ou être reliées ensemble. Les images peuvent être associées chacune à une indication alphanumérique.

Le dispositif peut également être associé à un ordinateur permettant d'afficher des images de référence.

Le dispositif d'observation peut, par ailleurs, comporter ou être utilisé en combinaison avec un outil de plissement ou d'étirement de la peau.

Cet outil peut comporter, par exemple, deux parties destinées à être appliquées sur la peau et pouvant être écartées ou rapprochées pour étirer ou plisser la peau.

L'outil peut comporter sur chaque partie destinée à être appliquée sur la peau une bande d'un adhésif double face lui permettant d'adhérer à la peau.

L'outil peut être fixé sur le dispositif d'observation.

Au moins une différence entre les images observées au travers du dispositif d'observation peut être le degré de plissement ou d'étirement de la peau.

Les deux parties peuvent être rapprochées contre l'action de l'organe élastique puis posées sur la peau et relâchées. L'organe élastique peut les solliciter alors en écartement ou assister l'utilisateur qui les écarte manuellement.

La liaison qui relie les deux parties peut être configurée de manière à permettre à l'utilisateur de les écarter d'une distance prédéterminée. La liaison peut comporter par exemple au moins une butée déterminant l'écartement maximal des deux parties. La liaison peut être configurée de manière à créer un point dur dans le déplacement des deux parties lorsque celles-ci atteignent leur écartement maximum. La liaison peut par exemple comporter au moins un organe de verrouillage qui s'enclenche lorsque l'écartement est maximal.

Chacune des deux parties peut présenter par exemple une forme générale en U lorsque l'outil est observé de dessus, dans une direction perpendiculaire à la direction de déplacement relatif de deux parties.

Ces dernières peuvent être reliées entre elles par deux tiges, l'une des tiges étant fixée sur l'une des parties et l'autre tige fixée sur l'autre partie, chaque tige qui est fixée dans l'une des parties pouvant coulisser dans un logement de l'autre partie. Ce logement peut traverser une branche du U.

Les deux parties peuvent être configurées de manière à former entre elles, lorsqu'elles sont en position rapprochée, une fenêtre d'observation de la peau. Dans le cas où les deux parties présentent chacune une forme en U, cette fenêtre est formée par les deux concavités du U.

Un matériau permettant de réaliser une empreinte, par exemple une cire ou une résine, peut être coulé dans la fenêtre précitée dans chacune des positions rapprochée ou écartée des deux parties de l'outil, par exemple afin de comparer l'aspect de la peau à l'état étiré ou non étiré à des intervalles de temps successifs.

L'outil d'étirement de la peau peut être utilisé notamment pour faire apparaître des microkystes ou d'autres défauts de la peau.

L'outil d'étirement peut encore être configuré de manière à permettre de mesurer l'écartement entre les deux parties appliquées sur la peau sous la seule action de l'organe élastique. Un écartement plus ou moins grand des deux parties peut traduire une capacité d'étirement plus ou moins grande de la peau. Dans le cas où la liaison entre les parties comporte des tiges comme défini plus haut, l'une des tiges précitée peut comporter des graduations permettant à l'utilisateur d'effectuer une mesure de l'écartement entre les deux parties.

L'invention a encore pour objet un procédé pour évaluer le degré d'une caractéristique de la typologie corporelle, caractérisé par le fait qu'il comporte l'étape suivante :
- observer la peau ou les cheveux au moyen d'un dispositif d'observation tel que défini plus haut.

Les images observées peuvent être comparées avec des images de référence, afin de sélectionner l'une d'elles.

Dans une mise en oeuvre particulière du procédé, on observe le contraste entre la lumière qui comporte une composante de réflexion et une composante rétrodiffusée et la lumière qui comporte essentiellement une composante rétrodiffusée, en éclairant la zone examinée avec une lumière polarisée et en l'observant avec un analyseur.

Les images de référence peuvent être affichées à l'écran d'un ordinateur, comme indiqué plus haut.

Les images de référence peuvent notamment être transmises à l'ordinateur depuis un serveur par un réseau informatique, avant d'être affichées sur ledit écran.

Le procédé peut comporter en outre l'étape consistant à transmettre au serveur une indication représentative de l'image de référence sélectionnée. Le serveur peut alors être programmé de manière à établir un diagnostic, par exemple, ou préconiser un produit cosmétique ou de soins.

Dans une mise en oeuvre particulière de l'invention, on procède à une évaluation d'une caractéristique de la typologie corporelle, on effectue un traitement, notamment cosmétique, susceptible d'avoir une incidence sur ladite caractéristique, puis on procède à une nouvelle évaluation pour détecter une éventuelle évolution de ladite caractéristique et déterminer l'efficacité du traitement.

Comme indiqué plus haut, on peut effectuer à l'aide du même dispositif d'observation, successivement ou simultanément, au moins deux types d'observations, choisis parmi les suivants :
i) observation sous éclairage diffus,
ii) observation sous éclairage rasant,
iii) observation sous éclairage directif,
iv) observation par transillumination,
v) observation sous lumière polarisée avec un analyseur non croisé,
vi) observation sous lumière polarisée avec un analyseur croisé,
vii) observation en comprimant la peau,
viii) observation sans comprimer la peau,
ix) observation en étirant la peau,
x) observation sans étirer la peau,
xi) observation en plissant la peau,
xii) observation sans plisser la peau.

Les types d'observation choisis peuvent notamment être i) et ii), v) et vi), vii) et viii), ix) et x) ou xi) et xii).

Dans le cas de l'observation selon ix) et x) ou xi) et xii), le procédé peut comporter l'étape consistant à, avant d'observer une zone de peau à l'aide du dispositif décrit plus haut, appliquer un outil d'étirement ou de plissement de la peau, par exemple un outil tel que défini précédemment, sur la zone de peau et étirer ou plisser la zone de peau.

L'invention a encore pour objet, selon un autre de ses aspects, un procédé pour évaluer le degré d'une caractéristique de la typologie corporelle, pouvant comporter les étapes suivantes :
- appliquer un outil d'étirement ou de plissement de la peau sur celle-ci,
- éventuellement prendre une empreinte de la zone de peau avant étirement ou plissement,
- étirer ou plisser la zone de peau,
- prendre une nouvelle empreinte de la zone de peau,
- observer la ou les empreintes ainsi obtenues à l'aide du dispositif d'observation défini plus haut.

D'autres caractéristiques et avantages de la présente invention ressortiront à la lecture de la description détaillée qui va suivre, d'exemples non limitatifs de mise en oeuvre, et à l'examen du dessin annexé, sur lequel :
- la figure 1 représente de manière très schématique, en coupe axiale partielle, un instrument optique pour la mise en oeuvre de l'invention,
- la figure 2 est une vue partielle de dessus selon la flèche II de la figure 1, illustrant le positionnement des éléments lumineux,
- la figure 3 représente le dispositif de la figure 1 dans une configuration d'observation sous éclairage rasant,
- la figure 4 représente le dispositif de la figure 1 dans une configuration permettant une transillumination,
- la figure 5 est une coupe axiale, schématique et partielle, d'une variante du dispositif de la figure 1,
- la figure 6 représente le dispositif de la figure 5, dans une configuration permettant un éclairage rasant,
- la figure 7 représente le dispositif de la figure 5 dans une configuration permettant la transillumination,
- la figure 8 représente le polariseur du dispositif des figures 5 à 7, en vue de dessus,
- la figure 9 est une vue partielle de dessus, représentant le disque analyseur du dispositif des figures 5 à 7, et illustrant la disposition des émetteurs lumineux,
- la figure 10 représente une variante du dispositif des figures 5 à 7,
- la figure 11 représente, en coupe axiale schématique et partielle, un autre exemple d'instrument optique pour la mise en oeuvre de l'invention,
- la figure 12 est une vue partielle de dessus de la figure 11,
- la figure 13 représente le dispositif de la figure 11 dans une configuration d'éclairage rasant,
- la figure 14 représente le dispositif de la figure 11 dans une configuration permettant la transillumination,
- la figure 15 représente un autre exemple de dispositif, en perspective éclatée,
- la figure 16 est une vue partielle de face selon XVI de la figure 15,
- la figure 17 représente une variante d'une partie du dispositif de la figure 15,
- la figure 18 représente de manière très schématique un atlas de brillance,
- la figure 19 représente de manière très schématique un atlas de contrastes,
- la figure 20 représente de manière très schématique un atlas de sécheresse cutanée,
- la figure 21 illustre la réalisation d'un atlas sous la forme d'un ensemble d'images reliées,
- la figure 22 représente de manière schématique un ordinateur permettant d'afficher, sur un écran, des images de référence,
- la figure 23 illustre la connexion d'un ordinateur, sur lequel peuvent être affichées des images de référence, à un serveur distant,
- les figures 24 et 25 représentent en vue de dessus schématique un outil de plissement de la peau avec les parties d'application sur la peau respectivement en positions écartée et rapprochée,
- les figures 26 et 27 sont des coupes partielles d'un outil d'étirement de la peau, lorsque l'outil est en configuration de mise en place sur la peau et d'étirement de la peau, et
- la figure 28 représente partiellement une variante de l'outil des figures 24 à 27.

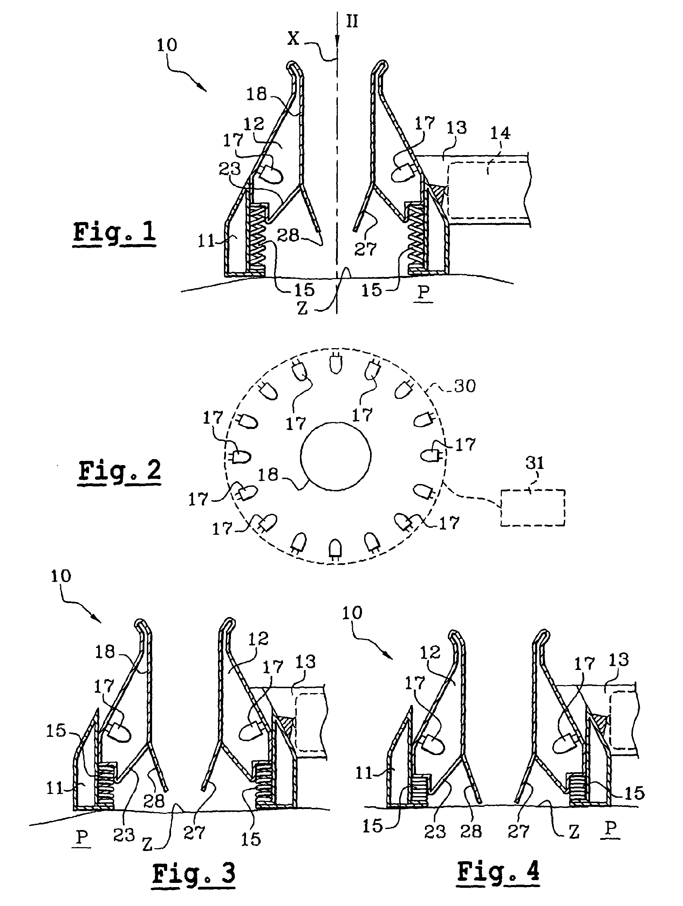

L'instrument optique 10 représenté sur les figures 1 à 4 est destiné à l'observation de la peau P ou des cheveux et comporte un support 11 à poser sur la surface à observer, un ensemble mobile 12 pouvant être déplacé par rapport au support 11 selon un axe X, et une poignée 13 permettant la préhension de l'instrument par un utilisateur, cette poignée 13 étant fixe par rapport au support 11 et logeant intérieurement une ou plusieurs piles ou accumulateurs 14.

Un système de rappel élastique interagit entre le support 11 et l'ensemble mobile 12, de manière à rappeler ce dernier dans une position de repos, correspondant à la figure 1, dans laquelle l'ensemble 12 vient en appui contre la poignée 13. Dans l'exemple illustré, ce système de rappel comporte plusieurs ressorts 15 en travaillant en compression, venant en appui à une extrémité contre un épaulement du support 11 et à l'autre extrémité contre l'ensemble 12.

Ce dernier comporte une pluralité d'émetteurs lumineux 17 constitués, dans l'exemple décrit, par des diodes électroluminescentes blanches, réparties tout autour de l'axe X, comme illustré sur la figure 2, de manière à produire soit un éclairage diffus de la zone Z examinée lorsque tous les émetteurs lumineux sont alimentés, soit un éclairage dans une ou plusieurs directions particulières, si seulement une partie des émetteurs lumineux est alimentée. Les émetteurs lumineux 17 sont alimentés en électricité grâce aux piles 14 par un circuit d'alimentation 30, représenté très schématiquement, dans un souci de clarté du dessin. Ce circuit d'alimentation 30 est relié à un circuit de commande 31.

L'observation s'effectue au travers d'un tube 18 de l'ensemble mobile 12.

Un élément optique 23 est placé sur le trajet de la lumière entre les émetteurs lumineux 17 et la zone examinée Z. Cet élément optique 23 est constitué dans l'exemple considéré par une pièce annulaire translucide, qui peut être incolore ou colorée, et qui constitue un diffuseur.

Le tube 18 est prolongé inférieurement par un écran 27, dont le bord libre 28 délimite le champ d'observation.

L'instrument optique 10 permet d'effectuer des observations successives, dans des conditions d'éclairage différentes, de la même surface.

Dans la configuration représentée à la figure 1, la zone examinée Z peut être éclairée de manière diffuse et omnidirectionnelle par la totalité des émetteurs lumineux 17.

L'écran 27 peut être rapproché de la zone examinée Z grâce à la mobilité de l'ensemble 12 par rapport au support 11, comme illustré sur la figure 3. Sur cette figure, on voit que le bord inférieur 28 de l'écran 27 a pu être rapproché par l'utilisateur en exerçant une pression suffisante vers le bas sur l'ensemble 12, contre l'action de rappel des ressorts 15. On peut alors obtenir un éclairage rasant de la zone examinée Z, capable de faire ressortir davantage le relief de la peau ou des cheveux et d'augmenter la brillance.

L'utilisateur a également la possibilité d'appuyer complètement sur l'ensemble 12 de manière à amener l'écran 27 au contact de la surface de la peau ou des cheveux, comme illustré sur la figure 4. Dans ce cas, la zone examinée Z est éclairée uniquement par transillumination, c'est-à-dire par diffusion de la lumière dans le tissu observé, ce qui permet par exemple d'obtenir une information sur sa transparence, sa couleur ou son irrigation par le sang. Le courant d'alimentation des émetteurs lumineux 17 peut rester inchangé entre les différentes observations.

Le circuit de commande 31 peut être constitué par un simple interrupteur électrique commandant l'alimentation en tout ou rien de la totalité des émetteurs lumineux 17 ou par un système plus complexe, permettant par exemple d'alimenter sélectivement certains émetteurs lumineux 17, afin d'obtenir par exemple un éclairage directionnel ou pluridirectionnel, fixe ou tournant, voire dans le cas où les émetteurs lumineux comporteraient des diodes de différentes couleurs ou capables d'émettre à des longueurs d'ondes variables, pour modifier les caractéristiques spectrales de la lumière venant éclairer la zone examinée.

Conformément à l'invention, entre les différentes configurations d'observation de la zone examinée représentées sur les figures 1, 3 et 4, au moins une caractéristique d'observation autre que le grossissement ou l'intensité de la source lumineuse intégrée a été modifiée.

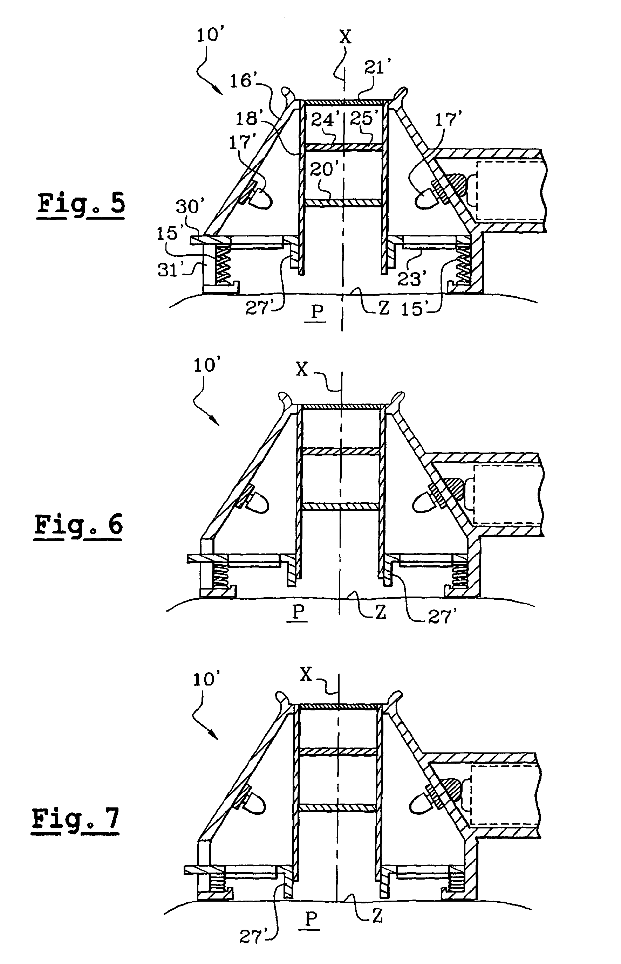

On a représenté sur les figures 5 à 7 un instrument optique 10' qui correspond à une variante de l'instrument 10 décrit précédemment.

Dans cette variante, l'instrument 10' comporte un corps 16' qui est fixe et un écran 27' qui est mobile par rapport au corps 16' selon l'axe X, pour modifier les conditions d'illumination de la zone observée Z, afin de permettre par exemple de passer d'un éclairage diffus multidirectionnel à un éclairage rasant, comme illustré à la figure 6, voire à un éclairage par transillumination, comme illustré sur la figure 7.

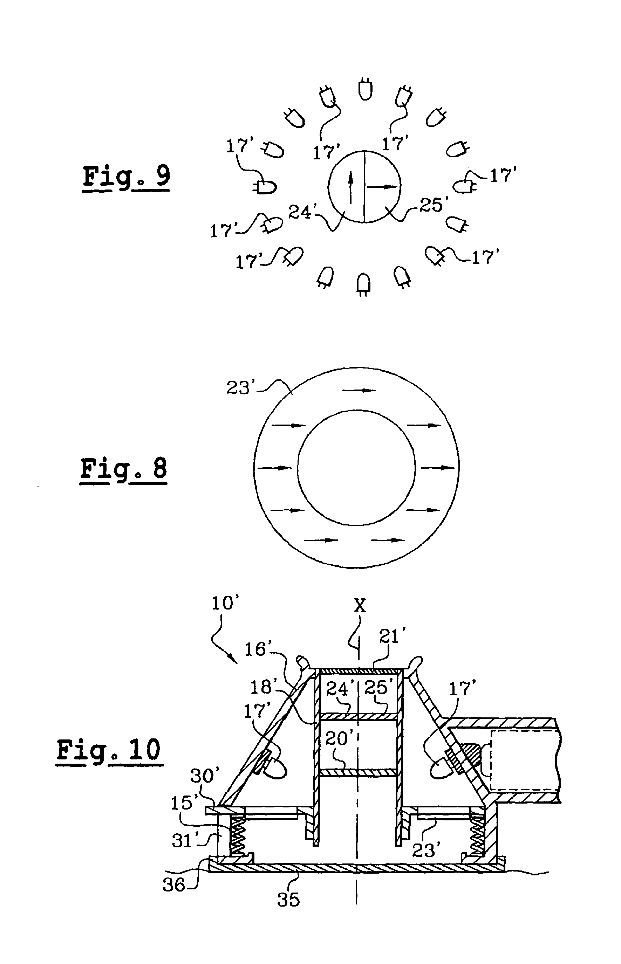

Un polariseur 23' représenté isolément sur la figure 8 est placé sur le trajet de la lumière entre les émetteurs lumineux 17' et la zone observée Z. Ce polariseur 23' présente une forme annulaire et une direction de polarisation unique, indiquée par des flèches sur la figure 8.

L'écran 27' est monté coulissant sur un tube 18' qui supporte des lentilles 20', 21' et des polariseurs 24', 25' et peut être déplacé grâce à une patte 30' faisant saillie à l'extérieur de l'instrument, traversant une fente 31' du corps 16', sur lequel sont montés les émetteurs lumineux 17'. Ces derniers sont agencés de la même manière que les émetteurs lumineux 17 de l'ensemble précédent. Un avantage de l'instrument 10' est de ne pas modifier la distance entre la zone examinée et les lentilles 20', 21' lors du changement de configuration de l'éclairage par déplacement de l'écran 27'.

Dans l'exemple considéré, deux polariseurs 24' et 25', en forme chacun de demi-disque et réunis par leur base de manière à former un disque analyseur, sont placés sur le trajet de la lumière dans le tube 18', dans le plan focal image de la lentille 20'. Les directions de polarisation des polariseurs 24' et 25' sont perpendiculaires entre elles et indiquées par des flèches sur la figure 9.

L'analyseur constitué par les polariseurs 24' et 25' permet d'obtenir une demi-image avec brillance et une demi-image sans brillance, en choisissant la direction de polarisation de l'un des demi-disques 24' et 25' parallèle à la direction de polarisation du polariseur 23'.

Le cas échéant, les polariseurs 24' et 25' sont remplacés par un polariseur monté rotatif autour de l'axe X afin de pouvoir faire d'une part coïncider sa direction de polarisation avec la direction de polarisation de la lumière incidente et d'autre part orienter ensuite sa direction de polarisation perpendiculairement à la direction de polarisation du polariseur 23'.

L'analyseur en deux parties 24', 25' permet cependant d'obtenir une image contrastée, et donc de profiter de la sensibilité accrue de l'oeil humain à un contraste.

Chaque instrument 10 ou 10' peut recevoir, comme illustré pour l'instrument 20' sur la figure 10, une vitre 35 à travers laquelle s'effectue l'observation. Cette vitre 35 peut être réalisée en matière plastique par exemple, et comporter un rebord 36 permettant son positionnement sur l'instrument. Dans l'exemple considéré, la vitre 35 constitue un accessoire amovible mais on ne sort pas du cadre de la présente invention lorsque la vitre est escamotable et reste solidaire de l'instrument dans sa position escamotée. Lorsqu'elle est en place sur l'instrument, la vitre 35 permet de comprimer la peau et d'en chasser le sang ; on peut ainsi effectuer des observations de la peau en diminuant l'incidence de la couleur du sang sur la couleur de la peau. On peut procéder à une série d'observations sans la vitre 35, puis à une nouvelle série d'observations avec la vitre 35, sous différents types d'éclairage ou de polarisations et comparer les résultats pour en tirer une information utile.

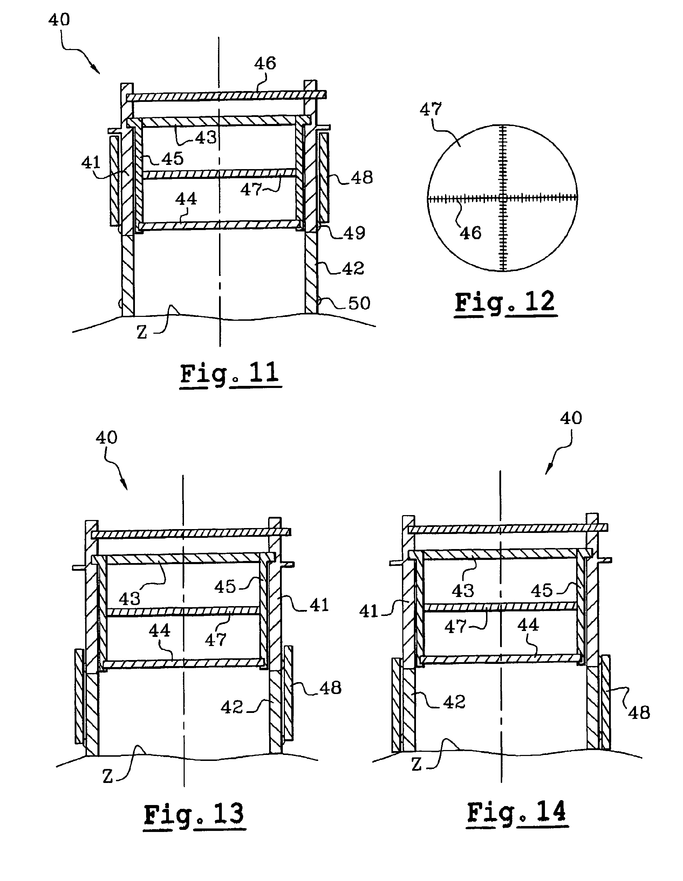

On a représenté sur les figures 11, 13 et 14 un instrument 40 dont une particularité, par rapport aux instruments 10 et 10' précédemment décrits, est de ne comporter aucun éclairage intégré.

L'instrument 40 comporte un corps 41 prolongé vers le bas par une jupe transparente 42.

Le corps 41 sert de support à un ensemble optique comportant des lentilles 43, 44 portées par un tube 45, encliqueté dans le corps 41. L'ensemble optique comporte également un réticule 46 permettant de mesurer une distance sur la zone examinée. Ce réticule est imprimé, dans l'exemple décrit et représenté isolément sur la figure 12, sur un verre 47 placé sur le trajet de la lumière provenant de la zone examinée.

Un filtre coloré 46 est monté de manière amovible sur le corps 41. Dans l'exemple représenté, ce filtre est bleu, de manière à faire ressortir, si on le souhaite, les tâches pigmentaires.

Un écran constitué par une bague opaque 48 peut coulisser sur le corps 41 et sur la jupe transparente 42, entre une position complètement escamotée, correspondant à la figure 11, dans laquelle la bague 48 ne vient pas recouvrir la jupe transparente 42, une position intermédiaire correspondant à la figure 13, dans laquelle la bague 48 vient recouvrir une majeure partie de la jupe transparente 42, afin de permettre un éclairage rasant de la zone examinée, et une position complètement descendue de la bague 48 correspondant à la figure 14, dans laquelle cette dernière recouvre en totalité la jupe transparente 42 et permet un éclairage par transillumination. Dans l'exemple représenté, des godrons 49 et 50 sont formés sur le corps 41 et la jupe transparente 42, de manière à permettre l'immobilisation de la bague 48 dans chacune des différentes configurations décrites.

Lorsque la zone examinée est observée par transillumination, l'observateur peut mesurer au moyen du réticule la distance à partir de laquelle la diffusion de la lumière n'est plus visible et en tirer une information sur la transparence de la peau.

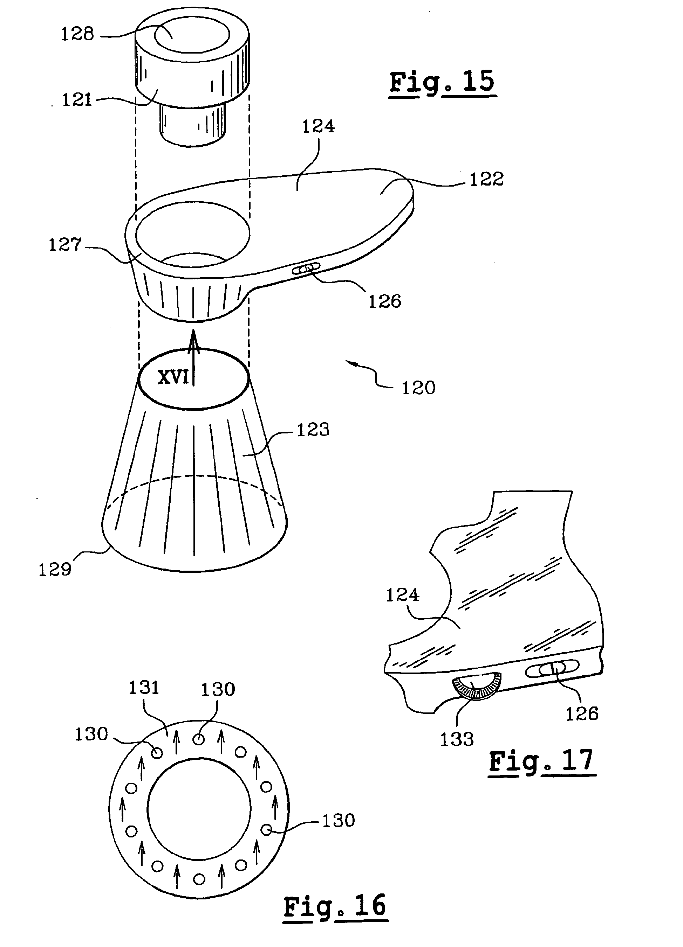

On a représenté à la figure 15 un autre exemple de dispositif d'observation 120 réalisé conformément à l'invention.

Ce dispositif 120 comporte une source lumineuse 122, un embout 123, et un ensemble optique 121.

La source lumineuse 122 comporte une poignée 124 qui forme un logement à l'intérieur duquel sont disposées une ou plusieurs piles ou accumulateurs, et une partie ajourée 127 dans laquelle peut être engagé l'ensemble optique 121.

Ce dernier comporte un oculaire 128 et une ou plusieurs lentilles non représentées, de manière à produire une image agrandie, ainsi qu'un polariseur.

La source lumineuse 122 comporte un sélecteur 126 permettant d'éclairer la zone à observer avec par exemple deux intensités lumineuses différentes ou deux types d'éclairage différents, par exemple l'un simulant la lumière du jour et l'autre un éclairage incandescent.

L'embout 123 présente une forme générale tronconique avec une base 129 ayant un diamètre supérieur au champ d'observation de l'ensemble optique 121.

Dans l'exemple illustré, le diamètre de la base 129 est de l'ordre de 40 mm et celui du champ d'observation de l'ordre de 30 mm. On évite ainsi que la pression de contact de l'embout 123 sur la peau influence l'aspect de la zone située dans le champ d'observation.

On peut prévoir une fixation magnétique de l'embout sur la source lumineuse 122. L'embout 123 peut par exemple comporter un anneau aimanté métallique.

L'embout 123 peut comporter à son extrémité qui vient au contact de la peau une bague amovible.

La source lumineuse 122 comporte, comme on peut le voir sur la figure 16, une pluralité de diodes électroluminescentes 130 dont l'axe est sensiblement parallèle à celui de l'ensemble optique 121. Ces diodes électroluminescentes 130 sont recouvertes par un polariseur 131.

La hauteur de l'embout 123 est choisie de telle sorte que l'éclairage de la zone de peau située dans le champ d'observation soit sensiblement homogène.

Le dispositif d'observation 120 s'utilise de la manière suivante :
L'embout 123 et l'ensemble optique 121 étant en place sur la source lumineuse 122, l'utilisateur peut faire tourner l'ensemble optique 121 relativement à la source lumineuse de telle sorte que le polariseur contenu dans l'ensemble optique 121 ait la même direction de polarisation que le polariseur 131 ou une direction sensiblement perpendiculaire.

Il est ainsi possible d'observer la peau successivement avec brillance et sans brillance.

Dans une variante de réalisation, l'ensemble optique 121 comporte deux polariseurs juxtaposés ayant des directions de polarisation perpendiculaires, à l'instar de l'exemple de réalisation décrit précédemment, en référence à la figure 9.

Le dispositif 120 peut comporter, comme illustré à la figure 17, une molette 133 permettant à l'utilisateur d'entraîner en rotation l'ensemble optique 121 avec la main qui tient la poignée 124.

Chaque image observée au moyen d'un instrument optique, par exemple l'un de ceux qui viennent d'être décrits, cette observation ayant eu lieu dans des conditions d'illumination spécifiques, peut être comparée avec une image de référence d'un atlas comportant plusieurs images de référence, par exemple des images exprimant à des degrés divers une caractéristique de la typologie corporelle, notamment la brillance ou la couleur de la peau, ou des images correspondant à divers degrés de contraste.

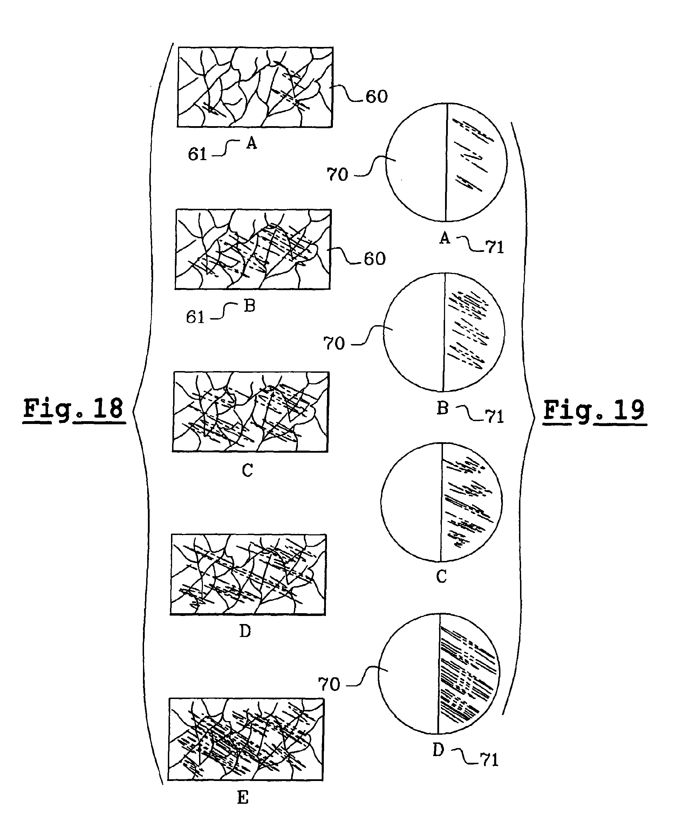

On a représenté de manière très schématique, à titre d'exemple, sur la figure 18, un atlas de brillance comportant plusieurs images de référence 60 correspondant chacune à un degré de brillance de la peau, quantifié par un identifiant alphanumérique 61.

On a représenté de manière très schématique sur la figure 19 un atlas de contraste ou d'écarts de luminosité ou de couleur, comportant plusieurs images de référence 70, correspondant à différents degrés de contraste ou d'écart pouvant être observés lors de l'utilisation d'un analyseur tel que celui formé par les demi-disques 24', 25', précédemment décrit. Ces images 70 sont associées à des identifiants alphanumériques 71, permettant de les repérer.

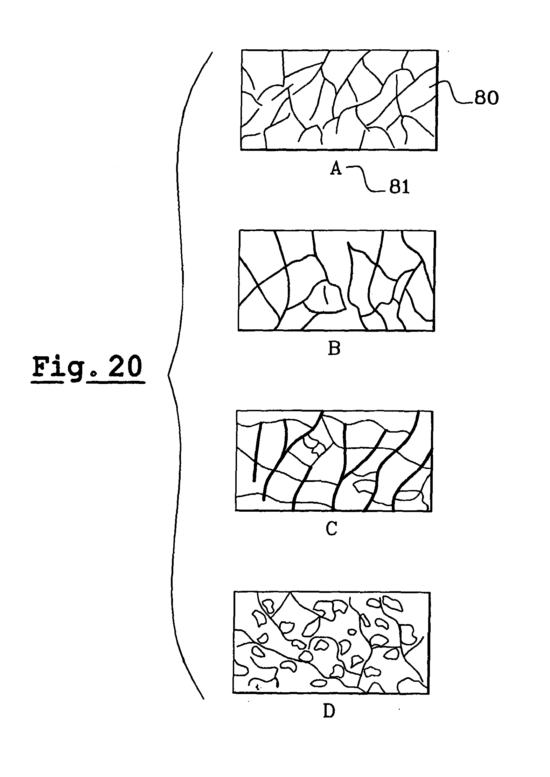

On a représenté à la figure 20 de manière très schématique un atlas de sécheresse cutanée, comportant plusieurs images 80, associées chacune à un identifiant alphanumérique 81, les images 80 exprimant divers degrés de sécheresse cutanée, allant d'une desquamation sévère, caractéristique d'une peau extrêmement sèche, à une absence de desquamation, traduisant une peau normale.

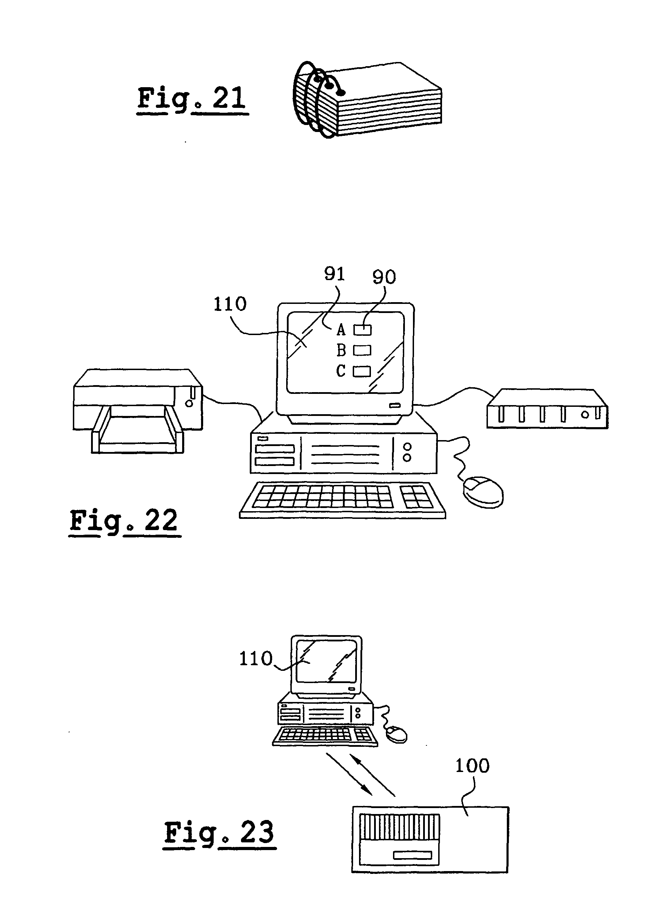

Les images de référence d'un atlas peuvent être imprimées sur un même support ou être reliées ensemble, comme illustré sur la figure 21.

Des images de référence peuvent également être affichées à l'écran d'un ordinateur 110, comme illustré à la figure 22, chaque image étant associée à un identifiant alphanumérique.

Les résultats d'une évaluation peuvent être transmis à distance par un réseau informatique, notamment le réseau Internet, au moyen de l'ordinateur 110 à un serveur 100, comme illustré à la figure 23. Le serveur 100 peut être agencé pour rendre un diagnostic en fonction des résultats transmis et, le cas échéant, préconiser un produit cosmétique ou de soins.

Dans le cas où des images de référence sont affichées sur l'écran d'un ordinateur, ces images peuvent être transmises par le serveur 100, après connexion de l'utilisateur sur le site Internet correspondant.

L'invention peut être mise en oeuvre de manière à suivre l'efficacité d'un traitement, en procédant à une évaluation après chaque phase du traitement et en comparant les résultats des évaluations successives.

Grâce à la possibilité d'obtenir au moyen d'un même instrument optique des informations de brillance, de couleur, de relief et de transparence, et grâce à l'utilisation d'un atlas permettant de quantifier ces informations, on peut constituer une banque de données multivectorielle regroupant un ensemble de vecteurs correspondant chacun à un individu, chaque vecteur comportant au moins deux composantes constituées chacune par le résultat d'une observation effectuée au moyen d'un même instrument optique.

Les dispositifs d'observation qui viennent d'être décrits peuvent être utilisés en combinaison avec un outil permettant de plisser ou d'étirer la peau.

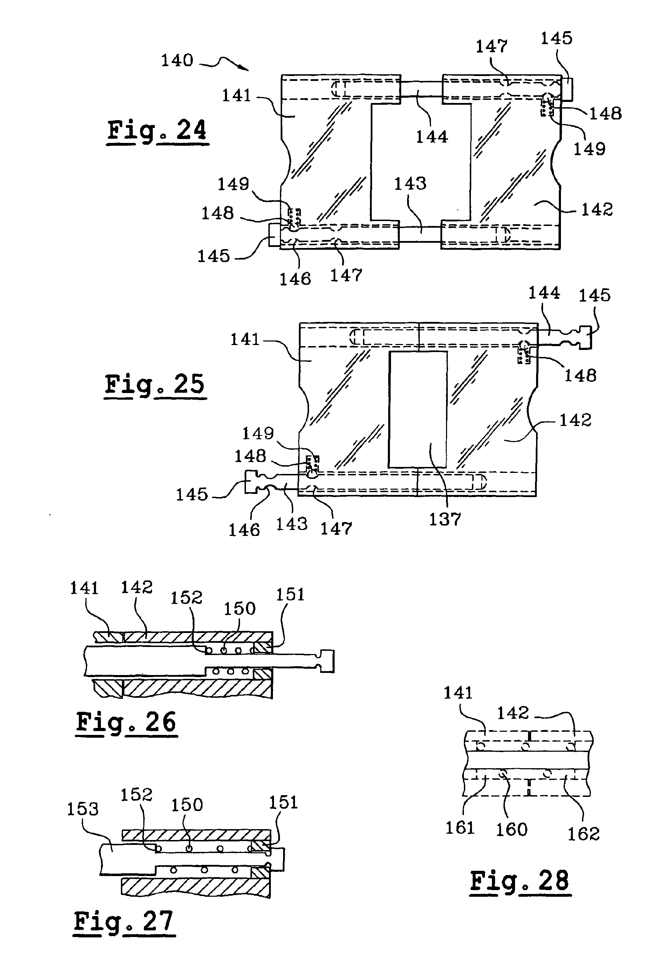

On a représenté sur les figures 24 et 25 un exemple d'outil 140 permettant de plisser ou d'étirer la peau.

L'outil 140 représenté sur ces figures comporte deux parties 141 et 142 destinées à être appliquées sur la peau et reliées par une liaison permettant un déplacement relatif de l'une par rapport à l'autre.

Dans l'exemple illustré, chacune des parties 141 et 142 présente une forme générale de U et les deux parties définissent entre elles, lorsque rapprochées, comme on peut le voir sur la figure 25, une fenêtre 137 d'observation de la peau, cette fenêtre étant formée par les concavités des U.

La liaison entre les parties 141 et 142 peut être réalisée de diverses manières.

Dans l'exemple considéré, la liaison comporte deux tiges 143 et 144 d'axes parallèles.

La tige 143 est fixée à une extrémité dans la partie 142 et peut coulisser dans un logement de la partie 141. La tige 143 présente à son extrémité opposée une tête élargie 145, se raccordant au reste de la tige en formant une gorge 146. La tige 143 comporte également une deuxième gorge 147 et la partie 141 comporte une bille 148, sollicitée par un ressort 149 en appui contre la tige 143.

Il en est de même de la tige 144, qui est fixe par rapport à la partie 141 et qui coulisse dans un logement de la partie 142.

Dans la position écartée des parties 141 et 142, chaque bille 148 vient s'engager dans la gorge 146 correspondante ce qui permet d'immobiliser les deux parties 141 et 142 dans leur position écartée. Lorsque les parties 141 et 142 sont rapprochées, chaque bille 148 s'engage dans la gorge 147 correspondante.

Cela permet de créer une sensation de point dur lorsque les deux parties sont en position écartée ou rapprochée.

L'outil 140 peut être positionné sur la peau en disposant un ruban adhésif double face sur chacune des parties 141 et 142.

On peut procéder, au moyen de l'un des dispositifs d'observation décrits précédemment, à deux observations, l'une lorsque la peau est non plissée, c'est-à-dire par exemple dès que l'outil 140 a été posé sur la peau dans la position écartée représentée à la figure 24, et l'autre après plissement de la peau, c'est-à-dire après que les parties 141 et 142 ont été rapprochées comme illustré sur la figure 25. Le dispositif d'observation peut être configuré de manière à coopérer avec l'outil 140.

On peut également procéder à une observation alors que la peau est à l'état non étiré, c'est-à-dire après que l'outil 140 a été posé sur la peau alors que les parties 141 et 142 sont à l'état rapproché, comme illustré sur la figure 25, et à une observation lorsque la peau est étirée, les parties 141 et 142 ayant été amenées dans la position écartée représentée à la figure 24.

Pour faciliter le passage de la position rapprochée représentée à la figure 25 à la position écartée représentée à la figure 24, l'outil 140 peut être avantageusement muni, comme illustré aux figures 26 et 27, d'au moins un organe élastique constitué par exemple d'un ressort hélicoïdal 150 engagé sur l'une des tiges. Chaque tige 143 ou 144 peut être munie de son ressort 150 correspondant. Le ressort 150 peut, comme illustré à la figure 26, prendre appui à une extrémité contre une bague 151 fixée sur l'une des parties destinée à être appliquée sur la peau et venir en appui à l'autre extrémité contre un épaulement 152 de la tige. Le ressort 150 est à l'état comprimé lorsque les deux parties 141 et 142 sont rapprochées. L'outil peut ainsi être posé sur la peau alors que les parties 141 et 142 sont rapprochées, chaque ressort 150 étant comprimé puis les parties 141 et 142 peuvent s'écarter sous l'effet de la détente des ressorts 150.

Au moins l'une des tiges 143 ou 144 peut comporter des graduations 153 qui deviennent apparentes lorsque les parties 141 et 142 s'écartent, ce qui permet de déterminer précisément l'écartement entre les deux parties 141 et 142.

On pourrait bien entendu, sans sortir du cadre de la présente invention, utiliser au moins un organe élastique disposé autrement, par exemple un ressort hélicoïdal 160 travaillant en compression, engagé sur l'une des tiges 143 et 144, dont les extrémités viennent en appui dans le fond de logements 161 et 162 se faisant face des parties 141 et 142, ces logements étant réalisés par exemple dans les branches des U, comme illustré à la figure 28.

Le dispositif de la figure 28 peut permettre, le cas échéant, de conserver les gorges 146, 147 et les billes 148 de l'exemple des figures 24 et 25.

Bien entendu, l'invention n'est pas limitée aux exemples qui viennent d'être décrits et l'on peut modifier la structure de l'instrument optique, notamment en remplaçant les diodes électroluminescentes par d'autres moyens d'éclairage, tels que par exemple des lampes à incandescence ou à fluorescence. Dans le cas où un écran serait utilisé pour observer par transillumination, cet écran peut être constitué par un élément rapporté, fixé de manière amovible sur l'instrument optique.

## Revendications

1. Dispositif portatif permettant d'évaluer au moins une caractéristique d'apparence de la peau ou des cheveux, le dispositif comportant des moyens permettant de générer au moins deux images d'une zone de la peau ou des cheveux à examinerée qui différent autrement que par le grossissement ou l'intensité d'une source lumineuse intégrée, le dispositif comportant :
- au moins une source lumineuse intégrée, ou
- étant agencé pour permettre l'éclairage de la zone examinée par la lumière naturelle,
le dispositif permettant, lorsqu'il est posé sur la zone de la peau ou des cheveux. d'observer directement ladite zone en la regardant au travers du dispositif, le dispositif étant agencé pour soumettre sélectivement la zone examinée à un éclairage diffus ou à un éclairage rasant

2. Dispositif selon la revendication précédente, **caractérisé par le fait qu'**il est agencé de telle sorte que lesdites images puissent être générées successivement en agissant entre deux observations sur un organe du dispositif permettant de faire varier une caractéristique autre que le grossissement ou l'intensité lumineuse de la source.

3. Dispositif selon la revendication 1, **caractérisé par le fait qu'**il est agencé de telle sorte que lesdites images puissent être générées simultanément, le dispositif étant agencé pour produire simultanément, avec des éléments optiques, deux images différentes de la zone observée.

4. Dispositif selon la revendication 1, **caractérisé par le fait qu'**il est agencé de telle sorte que lesdites images diffèrent par l'orientation d'une direction de polarisation d'un analyseur.

5. Dispositif selon la revendication 1, **caractérisé par le fait qu'**il est agencé de telle sorte que lesdites images différent par un degré de directivité de la lumière incidente.

6. Dispositif selon la revendication 1, **caractérisé par le fait qu'**il est agencé de telle sorte que lesdites images différent par la direction d'incidence de la lumière incidente.

7. Dispositif selon la revendication 1, **caractérisé par le fait qu'**il est agencé de telle sorte que lesdites images différent par la couleur de la lumière incidente.

8. Dispositif selon la revendication 1, **caractérisé par le fait qu'**il est agencé de telle sorte que lesdites images diffèrent par la pression appliquée sur la zone examinée.

9. Dispositif selon la revendication 6, **caractérisé par le fait qu'**il est agencé de telle sorte que lesdites images différent par le filtrage de la lumière provenant de la zone examinée.

10. Dispositif selon l'une quelconque des revendications précédentes, **caractérisé par le fait qu'**il comporte un système optique (20', 41' ; 44, 46 ; 121) agencé pour produire une image agrandie de la zone examinée.

11. Dispositif selon l'une quelconque des revendications précédentes, **caractérisé par le fait qu'**il est agencé pour pouvoir soumettre la zone examinée à un éclairage diffus.

12. Dispositif selon l'une quelconque des revendications précédentes, **caractérisé par le fait qu'**il est agencé pour pouvoir soumettre la zone examinée à un éclairage directif.

13. Dispositif selon l'une quelconque des revendications précédentes, **caractérisé par le fait qu'**il est agencé pour pouvoir soumettre la zone examinée à un éclairage rasant.

14. Dispositif selon l'une quelconque des revendications précédentes, **caractérisé par le fait qu'**il comporte au moins un écran (27 ; 27' ; 48) apte à être interposé entre une source de lumière et la zone examinée, de manière à pouvoir soumettre la zone examinée à un éclairage par transillumination.

15. Dispositif selon la revendication 14, **caractérisé par le fait que** l'écran est mobile, pouvant être déplacé d'une première position dans laquelle il est éloigné d'une surface bordant la zone examinée à une deuxième position dans laquelle il vient au contact de ladite surface.

16. Dispositif selon l'une des revendications 14 et 15, **caractérisé par le fait que** l'écran peut être déplacé et amené dans une position dans laquelle il permet un éclairage rasant de la zone examinée.

17. Dispositif selon l'une quelconque des revendications 14 à 16, **caractérisé par le fait que** l'écran comporte une paroi tubulaire (27 ; 27' ; 48) s'étendant, au cours de l'utilisation, autour de la zone examinée.

18. Dispositif selon la revendication 17, **caractérisé par le fait que** l'écran comporte une portion conique (27) ou pyramidale convergeant vers la zone examinée.

19. Dispositif selon l'une quelconque des revendications 14 à 18, l'écran (27 ; 27') étant mobile, **caractérisé par le fait qu'**il comporte au moins un ressort (15 15.') pour rappeler l'écran dans une position de repos en l'absence d'utilisation.

20. Dispositif selon l'une quelconque des revendications précédentes, **caractérisé par le fait qu'**il comporte au moins un filtre coloré (46).

21. Dispositif selon la revendication 20, **caractérisé par le fait que** le filtre (46) est de couleur bleue.

22. Dispositif selon l'une quelconque des revendications précédentes, **caractérisé par le fait qu'**il comporte au moins un polariseur (23'; 24' ; 131)

23. Dispositif selon la revendication précédente, **caractérisé par le fait qu'**il comporte au moins un polariseur (23' ; 131) placé sur le trajet de la lumière, entre une source de lumière et la zone examinée (Z).

24. Dispositif selon l'une les deux revendications immédiatement précédentes, **caractérisé par le fait qu'**il comporte au moins un polariseur (24'; 25') placé sur le trajet de la lumière entre la zone examinée et un oeil de l'observateur.

25. Dispositif selon l'une quelconque des revendications précédentes, **caractérisé par le fait qu'**il comporte au moins deux polariseurs (24', 25') d'orientations différentes, juxtaposés, placés sur le trajet de la lumière entre la zone examinée et un oeil de l'observateur.

26. Dispositif selon l'une quelconque des revendications précédentes, **caractérisé par le fait qu'**il comporte au moins un polariseur monté rotatif de manière à permettre à l'utilisateur de modifier l'orientation de sa direction de polarisation par rapport à une direction de référence.

27. Dispositif selon l'une quelconque des revendications précédentes, **caractérisé par le fait qu'**il est agencé pour permettre l'éclairage de la zone examinée par la lumière naturelle.

28. Dispositif selon la revendication précédente, **caractérisé par le fait qu'**il comporte une jupe (42) en matière plastique transparente, dont un bord peut s'étendre autour de la zone examinée.

29. Dispositif selon l'une quelconque des revendications 1 à 26, **caractérisé par le fait qu'**il comporte au moins une source lumineuse intégrée (17 ; 17' ; 122).

30. Dispositif selon la revendication 29, **caractérisée par le fait que** la source lumineuse intégrée comporte au moins un élément lumineux choisi parmi les suivants : lampe à incandescence, diode électroluminescente, lampe à fluorescence.

31. Dispositif selon la revendication 30, **caractérisé par le fait qu'**il comporte des éléments lumineux éclairant dans des gammes respectives de longueur d'onde différentes.

32. Dispositif selon l'une quelconque des revendications 29 à 31, **caractérisé par le fait qu'**il comporte plusieurs éléments lumineux et des moyens de commande (31) permettant d'alimenter sélectivement au moins une partie de ces éléments lumineux (17;17').

33. Dispositif selon l'une quelconque des revendications 29 à 32, **caractérisé par le fait qu'**il comporte des éléments lumineux (17 ; 17' ; 130) disposés circulairement.

34. Dispositif selon l'une quelconque des revendications 29 à 33, **caractérisé par le fait qu'**il comporte un logement (13) pour recevoir une ou plusieurs piles électriques (14).

35. Dispositif selon la revendication 34, **caractérisé par le fait que** le logement présente un axe sensiblement perpendiculaire; à une direction d'observation (X) de la zone examinée.

36. Dispositif selon l'une quelconque des revendications précédentes, **caractérisé par le fait qu'**il comporte une vitre (35) permettant de comprimer la peau dans la zone examinée pour en chasser le sang.

37. Dispositif selon la revendication précédente, **caractérisé par le fait que** ladite vitre constitue une accessoire amovible.

38. Dispositif selon l'une quelconque des revendications précédentes, **caractérisé par le fait qu'**il est associé à un atlas comprenant une pluralité d'images de référence (60 ; 70 ; 80).

39. Dispositif selon l'une quelconque des revendications 1 à 31, **caractérisé par le fait qu'**il est associé à un ordinateur (110) permettant d'afficher des images de référence (90).

40. Dispositif selon l'une quelconque des revendications précédentes, **caractérisé par le fait qu'**il comporte un réticule (46).

41. Dispositif selon l'une quelconque des revendications précédentes, **caractérisé par le fait qu'**il comporte un outil (140) de plissement ou d'étirement de la peau.

42. Procédé pour évaluer le degré d'une caractéristique de la typologie corporelle, **caractérisé par le fait qu'**il comporte les étapes suivantes :
- poser un dispositif (10; 10'; 40; 120) tel que défini dans l'une quelconque des revendications précédentes sur une zone de la peau ou des cheveux à examiner, et
- observer ladite zone au moyen du dispositif (10; 10';40; 120).

43. Procédé selon la revendication précédente, **caractérisé par le fait qu'**il comporte en outre les étapes suivantes :
- comparer l'image observée avec des images de référence (60 ; 70 ; 80 ; 90),
- sélectionner une image de référence.

44. Procédé selon la revendication précédente, **caractérisé par le fait qu'**on observe le contraste entre la lumière qui comporte une composante de réflexion et une composante rétrodiffusée et la lumière qui comporte essentiellement une composante rétrodiffusée, en éclairant la zone examinée avec une lumière polarisée et en observant avec un analyseur.

45. Procédé selon la revendication 43 ou 44, **caractérisé par le fait que** les images de référence (90) sont affichées à l'écran d'un ordinateur (110).

46. Procédé selon la revendication 45, **caractérisé par le fait que** les images de référence (90) sont transmises à l'ordinateur depuis un serveur (100) par un réseau informatique, avant d'être affichées sur ledit écran.

47. Procédé selon l'une quelconque des revendications 43 à 46, **caractérisé par le fait qu'**il comporte en outre l'étape consistant à transmettre au serveur (100) une indication représentative de l'image de référence sélectionnée.

48. Procédé selon l'une quelconque des revendications 42 à 47, **caractérisé par le fait que** l'on effectue à l'aide du même dispositif successivement ou simultanément au moins deux types d'observations, choisis parmi les suivants :
i) observation sous éclairage diffus,
ii) observation sous éclairage rasant,
iii) observation sous éclairage directif,
iv) observation par transillumination,
v) observation sous lumière polarisée avec un analyseur non croisé,
vi) observation sous lumière polarisée avec analyseur croisé,
vii) observation en comprimant la peau,
viii) observation sans comprimer la peau,
ix) observation en étirant la peau,
x) observation sans étirer la peau,
xi) observation en plissant la peau,
xii) observation sans plisser la peau.

49. Procédé selon l'une quelconque des revendications 42 à 48, **caractérisé par le fait que** l'on procède à une évaluation d'une caractéristique de la typologie corporelle, **par le fait que** l'on effectue un traitement, notamment cosmétique, susceptible d'avoir une incidence sur ladite caractéristique, puis **par le fait que** l'on procède à une nouvelle évaluation pour détecter une éventuelle évolution de ladite caractéristique et déterminer l'efficacité du traitement.

50. Procédé selon l'une quelconque des revendications 42 à 49, **caractérisé par le fait que** l'on observe par transillumination et **par le fait que** l'on mesure la distance à partir de laquelle la diffusion de la lumière n'est plus visible.

## Claims

1. A portable device enabling at least one characteristic of the appearance of the skin or the hair to be observed, the device comprising means enabling at least two images of a zone of the skin or the hair under examination to be generated, which images differ other than in magnification or in the intensity of an integrated light source, the device comprising:
- at least one integrated light source, or
- being arranged to enable the zone under examination to be illuminated by natural light,
the device enabling, when set on the zone of the skin or the hair to observe said zone directly by looking through the device,
the device being arranged to be capable of selectively subjecting the zone under examination to diffuse lighting or grazing lighting.

2. A device according to the preceding claim, **characterized by** the fact that it is arranged in such a manner that said images can be generated in succession by acting between two observations on a member of the device enabling a characteristic to be varied other than magnification or the intensity of the light source.

3. A device according to claim 1, **characterized by** the fact that it is arranged in such a manner that said images can be generated simultaneously, the device being arranged with optical elements to enable two different images of the zone under observation to be produced simultaneously.

4. A device according to claim 1, **characterized by** the fact that it is arranged in such a manner that said images differ in the orientation of a polarization direction of a polarization analyzer.

5. A device according to claim 1, **characterized by** the fact that it is arranged in such a manner that said images differ by a degree of directivity in the incident light.

6. A device according to claim 1, **characterized by** the fact that it is arranged in such a manner that said images differ by the direction of incidence of the incident light.

7. A device according to claim 1, **characterized by** the fact that it is arranged in such a manner that said images differ in the color of the incident light.

8. A device according to claim 1, **characterized by** the fact that it is arranged in such a manner that said images differ by the pressure applied to the zone under examination.

9. A device according to claim 6, **characterized by** the fact that it is arranged in such a manner that said images differ by the filtering of the light coming from the zone under examination.

10. A device according to any preceding claim, **characterized by** the fact that it possesses an optical system (20', 41'; 44, 46; 121) arranged to produce a magnified image of the zone under examination.

11. A device according to any preceding claim, **characterized by** the fact that it is arranged to be capable of subjecting the zone under examination to diffuse lighting.

12. A device according to any preceding claim, **characterized by** the fact that it is arranged to be capable of subjecting the zone under examination to directional lighting.

13. A device according to any preceding claim, **characterized by** the fact that it is arranged to be capable of subjecting the zone under examination to grazing light.

14. A device according to any preceding claim, **characterized by** the fact that it possesses at least one screen (27; 27'; 48) suitable for being interposed between a light source and the zone under examination so as to be capable of subjecting the zone under examination to lighting by transillumination.

15. A device according to claim 14, **characterized by** the fact that the screen is movable, being capable of being moved from a first position in which it is remote from a surface surrounding the zone under examination and a second position in which it comes into contact with said surface.

16. A device according to claim 14 or claim 15, **characterized by** the fact that the screen can be moved and brought into a position in which it enables the zone under examination to be illuminated by grazing light.

17. A device according to any one of claims 14 to 16, **characterized by** the fact that the screen comprises a tubular wall (27; 27'; 48) which, in use, extends around the zone under examination.

18. A device according to claim 17, **characterized by** the fact that the screen comprises a portion (27) that is conical or pyramid-shaped, converging towards the zone under examination.

19. A device according to any one of claims 14 to 18, in which the screen (27; 27') is movable, the device being **characterized by** the fact that it possesses at least one spring (15; 15') for urging the screen towards a rest position when not in use.

20. A device according to any preceding claim, **characterized by** the fact that it possesses at least one color filter (46).

21. A device according to claim 20, **characterized by** the fact that the filter (46) is blue in color.

22. A device according to any preceding claim, **characterized by** the fact that it possesses at least one polarizer (23'; 24'; 131).

23. A device according to the preceding claim, **characterized by** the fact that it possesses at least one polarizer (23'; 131) placed on the path of light between a light source and the zone (Z) under examination.

24. A device according to either one of the two immediately preceding claims, **characterized by** the fact that it possesses at least one polarizer (24'; 25') placed on the path of the light between the zone under examination and an observer's eye.

25. A device according to any preceding claim, **characterized by** the fact that it possesses at least two polarizers (24', 25') of different orientations that are juxtaposed and placed on the path of light between the zone under examination and an observer's eye.

26. A device according to any preceding claim, **characterized by** the fact that it possesses at least one pivotally-mounted polarizer so as to enable the user to modify the orientation of its direction of polarization relative to a reference direction.

27. A device according to any preceding claim, **characterized by** the fact that it is arranged to enable the zone under examination to be illuminated by natural light.

28. A device according to the preceding claim, **characterized by** the fact that it possesses a skirt (42) of transparent plastics material, the skirt having an edge suitable for extending around the zone under examination.

29. A device according to any one of claims 1 to 26, **characterized by** the fact that it possesses at least one integrated light source (17; 17'; 122).

30. A device according to claim 29, **characterized by** the fact that the integrated light source comprises at least one light emitting element selected from the following: an incandescent lamp, a light emitting diode, a fluorescent lamp.

31. A device according to claim 30, **characterized by** the fact that the optical instrument possesses light emitting elements emitting light in respective different wavelength ranges.

32. A device according to any one of claims 29 to 31, **characterized by** the fact that the optical instrument comprises a plurality of light emitting elements and control means (31) enabling at least a fraction of said light emitting elements (17; 17') to be powered selectively.

33. A device according to any one of claims 29 to 32, **characterized by** the fact that the optical instrument possesses light emitting elements (17; 17'; 130) disposed in a circle.

34. A device according to any one of claims 29 to 33, **characterized by** the fact that the optical instrument possesses a housing (13) for receiving one or more electric batteries (14).

35. A device according to claim 34, **characterized by** the fact that the housing presents an axis that is substantially perpendicular to an observation direction (X) for observing the zone under examination.

36. A device according to any preceding claim, **characterized by** the fact that the optical instrument possesses a pane (35) enabling the skin in the zone under examination to be compressed so as to expel blood therefrom.

37. A device according to the preceding claim, **characterized by** the fact that said pane constitutes a removable accessory.

38. A device according to any preceding claim, **characterized by** the fact that it is associated with an atlas containing a plurality of reference images (60; 70; 80).

39. A device according to any one of claims 1 to 31, **characterized by** the fact that it is associated with a computer (110) enabling reference images (90) to be displayed.

40. A device according to any preceding claim, **characterized by** the fact that it possesses a reticule (46).

41. A device according to any preceding claim, **characterized by** the fact that it possesses a tool (140) for creasing or stretching the skin.

42. A method of evaluating a degree of a characteristic of body typology, the method being **characterized by** the fact that it comprises the following steps:
. setting a device (10; 10'; 40; 120) as defined in any preceding claim on a zone of the skin or the hair under examination,
. observing said zone by means of the device (10; 10'; 40; 120).

43. A method according to the preceding claim, **characterized by** the fact that it further comprises the following steps:
. comparing the observed image with reference images (60; 70; 80; 90); and
.selecting a reference image.

44. A method according to the preceding claim, **characterized by** the fact that contrast is observed between light which comprises both a reflected component and a backscattered component and light which essentially comprises a backscattered component, by illuminating the zone under examination with polarized light and by observing it with a polarization analyzer.

45. A method according to claim 43 or 44, **characterized by** the fact that the reference images (90) are displayed on the screen of a computer (110).

46. A method according to claim 45, **characterized by** the fact that the reference images (90) are transmitted to the computer from a server (100) over a computer network prior to being displayed on said screen.

47. A method according to any one of claims 43 to 46, **characterized by** the fact that it further comprises the steps consisting in transmitting to the server (100) an indication representative of the selected reference image.

48. A method according to any one of claims 42 to 47, **characterized by** the fact that the same device is used successively or simultaneously to perform at least two types of observation selected from the following types:
i) observation under diffuse lighting;
ii) observation under grazing light;
iii) observation under directional lighting;
iv) observation by transillumination;
v) observation under polarized light using a non-crossed polarizer;
vi) observation under polarized light using a crossed polarizer;
vii) observation while compressing the skin;
viii) observation without compressing the skin;
ix) observation while stretching the skin;
x) observation without stretching the skin;
xi) observation while creasing the skin; and
xii) observation without creasing the skin.

49. A method according to any one of claims 42 to 48, **characterized by** the fact that a characteristic of body typology is evaluated, by the fact that treatment is performed, in particular cosmetic treatment likely to have an effect on said characteristic, and then by the fact that a new evaluation is performed in order to detect any change in said characteristic and to determine the effectiveness of the treatment.

50. A method according to any one of claims 42 to 49, **characterized by** the fact that observation is performed by transillumination and by the fact that the distance is measured beyond which light diffusion is no longer visible.

## Patentansprüche

1. Tragbare Vorrichtung, die die Bewertung von wenigstens einer Charakteristik der Erscheinung der Haut oder der Haare ermöglicht, wobei die Vorrichtung Mittel aufweist, die es ermöglichen wenigstens zwei Bilder eines Bereiches der Haut oder der Haare zu prüfen, die sich anders unterscheiden als durch die Vergrößerung oder die Intensität einer integrierten Licht- bzw. Leuchtquelle, wobei die Vorrichtung aufweist:
- mindestens eine integrierte Licht- bzw. Leuchtquelle, oder
- dass sie angeordnet ist, um die Beleuchtung des zu prüfenden Bereiches durch ein natürliches Licht zu ermöglichen,
wobei die Vorrichtung es ermöglicht, wenn sie auf dem Bereich der Haut oder der Haare liegt, den Bereich unmittelbar zu beobachten, wobei sie quer durch die Vorrichtung gesehen wird, wobei die Vorrichtung angeordnet ist, um den geprüften Bereich wahlweise einer diffusen Beleuchtung oder einer streifenden Beleuchtung auszusetzen.

2. Vorrichtung nach dem voranstehenden Anspruch, **dadurch gekennzeichnet, dass** diese in der Weise ausgebildet ist, dass die Bilder aufeinander folgend zwischen zwei Beobachtungen durch Einwirken auf einen Bestandteil der Vorrichtung, der es ermöglicht, eine Charakteristik zu variieren, die eine andere als die Vergrößerung oder die Intensität der Lichtquelle ist, erzeugt werden können.

3. Vorrichtung nach Anspruch 1, **dadurch gekennzeichnet, dass** diese in der Art ausgebildet ist, dass die Bilder gleichzeitig erzeugt werden können, wobei die Vorrichtung angeordnet ist, um gleichzeitig mit optischen Elementen zwei verschiedene Bilder des zu prüfenden Bereiches zu erzeugen,

4. Vorrichtung nach Anspruch 1, **dadurch gekennzeichnet, dass** diese in der Weise ausgebildet ist, dass sich die Bilder durch eine unterschiedliche Polarisationsrichtung eines Analysators unterscheiden.

5. Vorrichtung nach Anspruch 1, **dadurch gekennzeichnet, dass** sie in der Weise ausgebildet ist, dass sich die Bilder durch den Grad der Richtwirkung des einfallenden Lichtes unterscheiden.

6. Vorrichtung nach Anspruch 1, **dadurch gekennzeichnet, dass** diese in der Weise ausgebildet ist, dass sich die Bilder durch den Einfallswinkel des einfallenden Lichtes unterscheiden.

7. Vorrichtung nach Anspruch 1, **dadurch gekennzeichnet, dass** sie in der Weise ausgebildet ist, dass sich die Bilder durch die Farbe des einfallenden Lichtes unterscheiden.

8. Vorrichtung nach Anspruch 1, **dadurch gekennzeichnet, dass** sie in der Weise ausgebildet ist, dass sich die Bilder durch den auf den zur prüfenden Bereich angewandten Druck unterscheiden.

9. Vorrichtung nach Anspruch 6, **dadurch gekennzeichnet, dass** sie in der Weise ausgebildet ist, dans sich die Bilder durch die Filterung des Lichtes unterscheiden, das von dem geprüften Bereich ausgeht.

10. Vorrichtung nach irgendeinem der voranstehenden Ansprüche, **dadurch gekennzeichnet, dass** sie ein optisches System (20', 40'; 44, 46; 121) aufweist, das ausgebildet ist, um ein vergrößertes Bild des geprüften Bereiches zu erzeugen.

11. Vorrichtung nach irgendeinem der voranstehenden Ansprüche, **dadurch** gckennzeichnet, dass sie ausgebildet ist, um die geprüfte Zone einer diffusen Beleuchtung auszusetzen,

12. Vorrichtung nach irgendeinem der voranstehenden Ansprüche, **dadurch gekennzeichnet, dass** sie ausgebildet ist, um die geprüfte Zone einer gerichteten Beleuchtung auszusetzen.

13. Vorrichtung nach irgendeinem der voranstehenden Ansprüche, **dadurch gekennzeichnet, dass** sie ansgebildet ist, um den geprüften Bereich einer streifenden Beleuchtung auszusetzen,

14. Vorrichtung nach irgendeinem der voranstehenden Ansprüche, **dadurch gekennzeichnet, dass** diese mindestens eine Blende (27; 27'; 48) aufweist, die dazu geeignet ist, um zwischen der Lichtquelle und dem geprüften Bereich in der Weise angeordnet zu werden, um den geprüften Bereich einer Beleuchtung durch Durchleuchtung aussetzen.

15. Vorrichtung nach Anspruch 14, **dadurch gekennzeichnet, dass** die Blende beweglich ist, wobei sie von einer ersten Position, in der sie von einer Fläche entfernt ist, die den geprüften Bereich umrandet, zu einer zweiten Position versetzbar ist, in der sie mit der Fläche in Kontakt kommt.

16. Vorrichtung nach einem der Ansprüche 14 und 15, **dadurch gekennzeichnet, dass** die Blende verstellt und in eine Stellung geführt werden kann, in der sie eine streifende Beleuchtung der geprüften Zone erlaubt.

17. Vorrichtung nach irgendeinem der Ansprüche 14 bis 16, **dadurch gekennzeichnet, dass** die Blende eine röhrenförmige Seitenwand (27; 27'; 48) aufweist, die sich im Verlauf der Verwendung um die geprüfte Zone erstreckt.

18. Vorrichtung nach Anspruch 17, **dadurch gekennzeichnet, dass** die Blende einen konischen oder pyramidenförmigen Abschnitt (21) aufweist, der in Richtung des geprüften Bereichs konvergiert.

19. Vorrichtung nach einem der Anspruchs 14 bis 18, wobei die Blende (27; 27') beweglich ist, **dadurch gekennzeichnet, dass** sie mindestens eine Federung (15; 15') aufweist, um die Blende, wenn sie nicht gebraucht wird, in eine Ruhestellung zurückzuholen.

20. Vorrichtung nach einem der voranstehenden Ansprüche, **dadurch gekennzeichnet, dass** sie mindestens einen farbigen Filter (46) aufweiset.

21. Vorrichtung nach Anspruch 20, **dadurch gekennzeichnet, dass** der Filter (46) eine blaue Farbe hat.

22. Vorrichtung nach einem der voranstehenden Ansprüche, **dadurch gekennzeichnet, dass** sie mindestens einen Polarisator (23'; 24'; 131) aufweist.

23. Vorrichtung nach dem voranstehenden Anspruch, **dadurch gekennzeichnet, dass** sie mindestens einen Polarisator (23'; 131) aufweist, der auf dem Weg des Lichtes zwischen einer Lichtquelle und dem geprüften Bereich (Z) angeordnet ist.

24. Vorrichtung nach einem der zwei unmittelbar vorangehenden Ansprüche, **dadurch gekennzeichnet, dass** sie mindestens einen Polarisator (24'; 25') aufweist, der auf dem Weg des Lichtes zwischen dem geprüften Bereich und einem Auge des Beobachters angeordnet ist.

25. Vorrichtung nach irgendeinem der voranstehenden Ansprüche, **dadurch gekennzeichnet, dass** sie wenigstens zwei Polarisatoren (24', 25') mit unterschiedlicher Orientierung einander gegenüberliegend auf dem Weg des Lichtes zwischen dem geprüften Bereich und einem Auge des Beobachters aufweist.

26. vorrichtung nach einem der voranstehenden Ansprüche, **dadurch gekennzeichnet, dass** sie wenigstens einen Polarisator aufweist, der drehbar in einer Weise montiert ist, die es dem Benutzer erlaubt, die Orientierung seiner Polarisationsrichtung im Verhältnis zu einer Referenzrichtung zu verändern,

27. Vorrichtung nach einem der voranstehenden Ansprüche, **dadurch gekennzeichnet, dass** sie angeordnet ist, um die Beleuchtung des geprüften Bereichs durch natürliches Licht zu erlauben.

28. Vorrichtung nach dem voranstehenden Anspruch, **dadurch gekennzeichnet, dass** sie eine Verkleidung (42) einer Art aus durchsichtigem Kunststoff aufweist, von der ein Rand sich um den geprüften Bereich erstrecken kann.

29. Vorrichtung nach einem Ansprüche 1 bis 26, **dadurch gekennzeichnet, dass** sie wenigstens eine integrierte Lichtquelle (17;17'; 122) aufweist.

30. Vorrichtung nach Anspruch 29, **dadurch gekennzeichnet, dass** die integrierte Lichtquelle wenigstens ein Leuchtelement aufweist, das unter den folgenden ausgewählt ist: Glühlampe, Elektrolumineszenzdiode, Fluoreszenzleuchte.

31. Vorrichtung nach Anspruch 30, **dadurch gekennzeichnet, dass** sie in jeweiligen unterschiedlichen Bereichen der Wellenlänge leuchten.

32. Vorrichtung nach einem der Ansprüche 29 bis 31, **dadurch gekennzeichnet dass** sie mehrere Leuchtelemente und Steuermittel (31) aufweist, die es ermöglichten, wahlweise einen Teil der Leuchtelemente (17; 17') zu versorgen.

33. Vorrichtung nach einem der Ansprüche 29 bis 32, **dadurch gekennzeichnet, dass** sie Leuchtelemente (17; 17'; 130) aufweist, die kreisförmig angeordnet sind.

34. Vorrichtung nach einem der Ansprüche 29 bis 33, **dadurch gekennzeichnet, dass** sie einen Unterbringungsraum (13) aufweist, um ein oder mehrere elektrische Elemente (14) bzw. Quecksilberelemente aufzunehmen.

35. Vorrichtung nach Anspruch 34, **dadurch gekennzeichnet, dass** der Unterbringungsraum eine genau senkrechte Achse in einer Beobachtungsrichtung (X) zu dem geprüften Bereich darstellt.

36. Vorrichtung nach einem der voranstehenden Ansprüche, **dadurch gekennzeichnet, dass** sie eine Glasscheibe (35) aufweist, die es ermöglicht, die Haut in dem geprüften Bereich zu komprimieren, um das Blut herauszutreiben.

37. Vorrichtung nach dem voranstehenden Anspruch, **dadurch gekennzeichnet, dass** die Glasscheibe einen abnehmbaren Bestandteil bildet.

38. Vorrichtung nach einem der voranstehenden Ansprüche, **dadurch gekennzeichnet, dass** sie mit einem Atlas ausgebildet ist, der mehrere Referenzbilder (60; 70; 80) aufweist.

39. Vorrichtung nach einem Ansprüche 18 bis 31, **dadurch gekennzeichnet, dass** sie mit einem Computer (110) ausgestattet ist, der es erlaubt, Referenzbilder (90) deutlich zu zeigen.

40. Vorrichtung nach einem der voranstehenden Ansprüche, **dadurch gekennzeichnet, dass** sie ein Reticel bzw. Fadenkreuz (46) aufweist.

41. Vorrichtung nach einem der voranstehenden Ansprüche, **dadurch gekennzeichnet, dass** sie ein Gerät (140) zur Faltung oder zur Dehnung der Haut aufweist.

42. Verfahren zur Bewertung des Grades eines Charakteristikums der körperlichen Typologie, **dadurch gekennzeichnet, dass** es die folgenden Schritte aufweist:
- eine Vorrichtung (10; 10'; 40; 120) nach einem der voranstehenden Ansprüche wird auf einem Bereich der Haut oder der Haare, der zu prüfen ist, angeordnet, und
- der Bereich wird mittels der Vorrichtung (10; 10'; 40; 120) beobachtet.

43. Verfahren nach dem voranstehenden Anspruch, **dadurch gekennzeichnet, dass** es ferner die folgenden Schritte aufweise
- das beobachtete Bild wird mit Referenzbildern (60; 70; 80; 90) verglichen,
- ein Referenzbild wird ausgewählt.

44. Verfahren nach dem voranstehende Anspruch, **dadurch gekennzeichnet, dass** der Kontrast zwischen dem Licht, das eine Reflektionskomponente und eine rückstreuende Komponente aufweist, und dem Licht, das im wesentlichen eine rückstrenende Komponente aufweist, beim Bestrahlen des geprüften Bereichs mit einem polarisierten Licht und beim Beobachten mit einem Analysator beobachtet wird.

45. Verfahren nach Anspruch 43 oder 44, **dadurch gekennzeichnet, dass** die Referenzbilder (90) auf der Blende bzw. dem Bildschirm eines Computers (110) deutlich gezeigt werden.

46. Verfahren nach Anspruch 45, **dadurch gekennzeichnet, dass** die Referenzbilder (90) von einem Server (100) über ein informatisches Netzwerk übertragen werden, bevor sie auf der Blende bzw. dem Bildschirm deutlich dargestellt werden.

47. Verfahren nach einem der Ansprüche 43 bis 46, **dadurch gekennzeichnet, dass** es ferner den Schritt aufweist, der daraus besteht, an den Server (100) eine darstellende Anzeige des ausgewählten Referenzbildes zu übertragen.

48. Verfahren nach einem der Ansprüche 42 bis 47, **dadurch gekennzeichnet, dass** mit Hilfe der gleichen Vorrichtung aufeinander folgend oder gleichzeitig wenigstens zwei Arten von Beobachtungen bewirkt werden, die unter den folgenden ausgewählt sind:
i) Beobachten unter einer diffusen Beleuchtung,
ii) Beobachten unter einer streifenden Beleuchtung,
iii) Beobachten unter einer direkten Einstrahlung,
iv) Beobachten unter Durchleuchtung,
v) Beobachten unter polarisiertem Licht mit einem Analysator, der nicht quert bzw. kreuzt,
vi) Beobachten unter einem polarisierten Licht mit einem querenden bzw. streuzenden Analysator,
vii) Beobachten unter Komprimierung der Haut,
viii) Beobachten ohne die Haut zu Komprimieren,
ix) Beobachten unter Zugeinwirkung auf die Haut,
x) Beobachten ohne Zugeinwirkung auf die Haut,
xi) Beobachten unter Faltung der Haut,
xii) Beobachten ohne Faltung der Haut.

49. Verfahren nach einem der Ansprüche 42 bis 48, **dadurch gekennzeichnet, dass** eine Bewertung der Charakteristiken der körperlichen Topologie durchgeführt wird, und **dadurch**, dass eine Behandlung, insbesondere eine kosmetische Behandlung bewirkt wird, die geeignet ist, eine Einwirkung auf die Charakteristik zu haben und **dadurch**, dass eine neue Bewertung durchgeführt wird, um eine möglich Fortentwicklung der Charakteristik zu erfassen und die Wirksamkeit der Behandlung zu bestimmen.

50. Verfahren nach einem der Ansprüche 42 bis 49, **dadurch gekennzeichnet, dass** mittels Durchleuchtung beobachtet wird und **dadurch**, dass der Abstand gemessen wird, von dem aus die Durchgängigkeit des Lichtes nicht mehr sichtbar ist.
